# EUROPEAN PATENT SPECIFICATION

(11) **EP 3 342 447 B1**
(45) Date of publication and mention of the grant of the patent: **13.11.2019**
(21) Application number: 16889941.7
(22) Date of filing: 06.12.2016
(51) Int. Cl.: A61M 37/00, B65D 75/32, B65D 77/20, B65D 73/00

(54) **STORAGE SHEET AND MICRONEEDLE SHEET PACKAGING BODY**
AUFBEWAHRUNGSBLATT UND VERPACKUNGSKÖRPER FÜR MIKRONADELBLATT
FEUILLE DE STOCKAGE, ET EMBALLAGE POUR FEUILLE DE MICRO-AIGUILLE

(30) Priority: 11.02.2016 JP 2016024208
(43) Date of publication of application: 04.07.2018
(73) Proprietor: Nissha Co., Ltd., Kyoto-shi, Kyoto 604-8551 (JP)
(72) Inventor: MATSUZAKI, Tomohiro, Kyoto-shi Kyoto 604-8551 (JP); KARIYA, Masahiko, Kyoto-shi Kyoto 604-8551 (JP)
(74) Representative: Dilg, Haeusler, Schindelmann Patentanwaltsgesellschaft mbH
(86) International application number: PCT/JP2016/086264
(87) International publication number: WO 2017/138240

(56) References cited:
- WO-A1-2015/147030
- JP-A- 2015 181 895
- JP-A- 2015 181 895
- JP-U- S6 065 254
- JP-Y2- S5 411 256

## Description

### Technical Field

The present invention relates to a housing sheet and to a microneedle sheet package.

### Background Art

A microneedle sheet includes a plurality of microneedles arrayed on a sheet-like substrate. The microneedles each have a shape that produces almost no feeling of pain when the microneedles puncture the skin. The microneedle sheet is stuck to the skin to cause the microneedles to puncture the skin, and a wide range of drugs can be administered.

Since the microneedles are extremely delicate owing to their shape, there is a risk of breakage or splintering of the microneedles in the course of production, storage, shipping, transport and the like of the microneedle sheet. Thus, to prevent breakage or splintering of the microneedles, the present applicant has developed a method for producing a microneedle shapes of the microneedles are formed (for example, see Patent Literature 1). In this method, a stamper including protrusions having the same shapes as the shapes of the microneedles is pressed into a housing sheet to form the concave portions having the inverted shapes of the shapes of the microneedles. Then, a raw material of the microneedle sheet is poured into the concave portions, and a sticky sheet is stuck to a back face of the microneedle sheet. Finally, the microneedle sheet is hermetically sealed by using a sealing sheet.

### Citation List

### Patent Literature

Patent Literature 1: JP 2015-181895 A
Patent Literature 2: WO 2015147030

### Summary of Invention

### Technical Problem

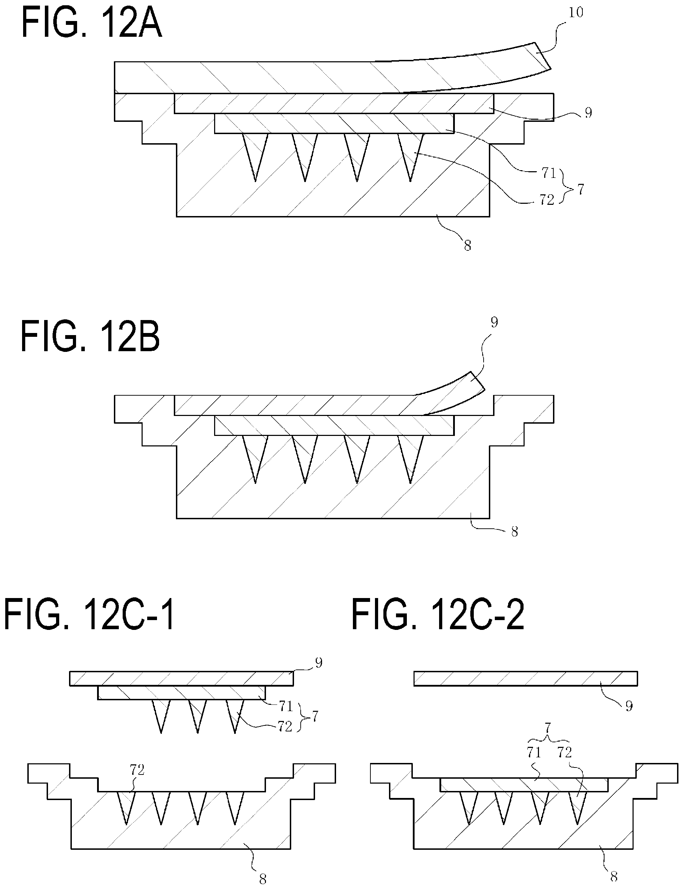

However, when a microneedle sheet as in the related art is used, a user peels a sealing sheet 10 (see FIG. 12A), and pulls off a microneedle sheet 7 from a housing sheet 8 while holding an edge of a sticky sheet 9 (see FIG. 12B). As a result, there has been a problem of remaining of some of microneedles 72 or the entire microneedle sheet 7 on the housing sheet 8 side (see FIGS. 12C-1 and 12C-2). The present invention is made to solve this problem, and an object of the present invention is to provide a housing sheet from which a microneedle sheet can be taken cleanly without remaining on the housing sheet side, and to provide a microneedle sheet package using such a housing sheet.

### Solution to Problem

A plurality of aspects as means for solving the problem will be described below. These aspects can optionally be combined as necessary.

A housing sheet of the present invention is a housing sheet including a housing portion for housing a microneedle sheet in which a plurality of microneedles are disposed on one face of a sheet-like substrate, wherein concave portions having inverted shapes of shapes of the microneedles are formed in the housing portion, and the housing portion includes a notch.

In a preferred aspect, the notch is a half-cut or a perforation.

A microneedle sheet package of the present invention includes: a microneedle sheet in which a plurality of microneedles are disposed on one face of a sheet-like substrate; a sticky sheet stuck entirely to the other face of the sheet-like substrate, and including a first excess portion jutting out from the sheet-like substrate along a peripheral shape of the sheet-like substrate; and a housing sheet including a housing portion in which concave portions having inverted shapes of shapes of the microneedles are formed, and in which the microneedle sheet is housed and in which a notch is formed, and including a face stuck to the first excess portion.

Furthermore, a microneedle sheet package of the present invention includes: a microneedle sheet in which a plurality of microneedles are disposed on one face of a sheet-like substrate; a sticky sheet stuck entirely to the other face of the sheet-like substrate, and including a first excess portion jutting out from the sheet-like substrate along a peripheral shape of the sheet-like substrate; a sealing sheet stuck entirely to a face opposite a face of the sticky sheet to which the sheet-like substrate is stuck, and including a second excess portion jutting out from the sticky sheet along a peripheral shape of the sticky sheet; and a housing sheet including a housing portion in which concave portions having inverted shapes of shapes of the microneedles are formed, and in which the microneedle sheet is housed and in which a notch is formed, and including a face stuck to the first excess portion and a face stuck to the second excess portion.

In a preferred aspect, the sealing sheet includes a metal layer laminated on a resin layer, a sticky layer laminated on a face stuck to the sticky sheet, and an adhesive layer laminated on the second excess portion.

In a preferred aspect, the notch is a half-cut or a perforation.

In these preferred aspects, an interior of the housing portion is empty.

### Advantageous Effects of Invention

A housing sheet according to the present invention is a housing sheet including a housing portion for housing a microneedle sheet in which a plurality of microneedles are disposed on one face of a sheet-like substrate, and is configured such that concave portions having inverted shapes of shapes of the microneedles are formed in the housing portion, and the housing portion includes a notch.

A microneedle sheet package of the present invention is configured to include a microneedle sheet in which a plurality of microneedles are disposed on one face of a sheet-like substrate, a sticky sheet stuck entirely to the other face of the sheet-like substrate, and including a first excess portion jutting out from the sheet-like substrate along a peripheral shape of the sheet-like substrate, and a housing sheet including a housing portion in which concave portions having inverted shapes of shapes of the microneedles are formed, and in which the microneedle sheet is housed and in which a notch is formed, and including a face stuck to the first excess portion.

Furthermore, a microneedle sheet package of the present invention is configured to include a microneedle sheet in which a plurality of microneedles are disposed on one face of a sheet-like substrate; a sticky sheet stuck entirely to the other face of the sheet-like substrate, and including a first excess portion jutting out from the sheet-like substrate along a peripheral shape of the sheet-like substrate; a sealing sheet stuck entirely to a face opposite a face of the sticky sheet to which the sheet-like substrate is stuck, and including a second excess portion jutting out from the sticky sheet along a peripheral shape of the sticky sheet; and a housing sheet including a housing portion in which concave portions having inverted shapes of shapes of the microneedles are formed, and in which the microneedle sheet is housed and in which a notch is formed, and including a face stuck to the first excess portion and a face stuck to the second excess portion.

Therefore, the present invention can provide a housing sheet from which a microneedle sheet can be taken cleanly without remaining on the housing sheet side, and a microneedle sheet package using such a housing sheet.

### Brief Description of Drawings

FIGS. 1A to 1C are cross-sectional views of a housing sheet according to an embodiment of the present invention.
FIGS. 2A to 2D are cross-sectional views illustrating an example of the case where a microneedle sheet is taken from the housing sheet of the present invention.
FIG. 3 is a cross-sectional view of a microneedle sheet according to an embodiment of the present invention.
FIG. 4 is a cross-sectional view illustrating a state where a sticky sheet is peeled from a housing sheet.
FIG. 5 is a cross-sectional view of a microneedle sheet according to another embodiment of the present invention.
FIG. 6 is a cross-sectional view illustrating a state where a sealing sheet is peeled from a housing sheet.
FIGS. 7A to 7D-2 are cross-sectional views illustrating an example of a method for producing the microneedle sheet package of the present invention.
FIGS. 8A to 8D are cross-sectional views illustrating another example of a method for producing the microneedle sheet package of the present invention.
FIGS. 9A to 9C are perspective views illustrating an example of the case where a microneedle sheet is used.
FIGS. 10A and 10B are cross-sectional views illustrating another example of the case where a microneedle sheet is used.
FIGS. 11A and 11B are cross-sectional views illustrating another example of the case where a microneedle sheet is used.
FIGS. 12A to 12C-2 are cross-sectional views for explaining the related art.

### Description of Embodiments

First, an example of a housing sheet according to an embodiment of the present invention will be described.

The housing sheet of the present invention is a housing sheet including a housing portion for housing a microneedle sheet in which a plurality of microneedles are disposed on one face of a sheet-like substrate, wherein concave portions including inverted shapes of shapes of the microneedles are formed in the housing portion, and the housing portion includes a notch.

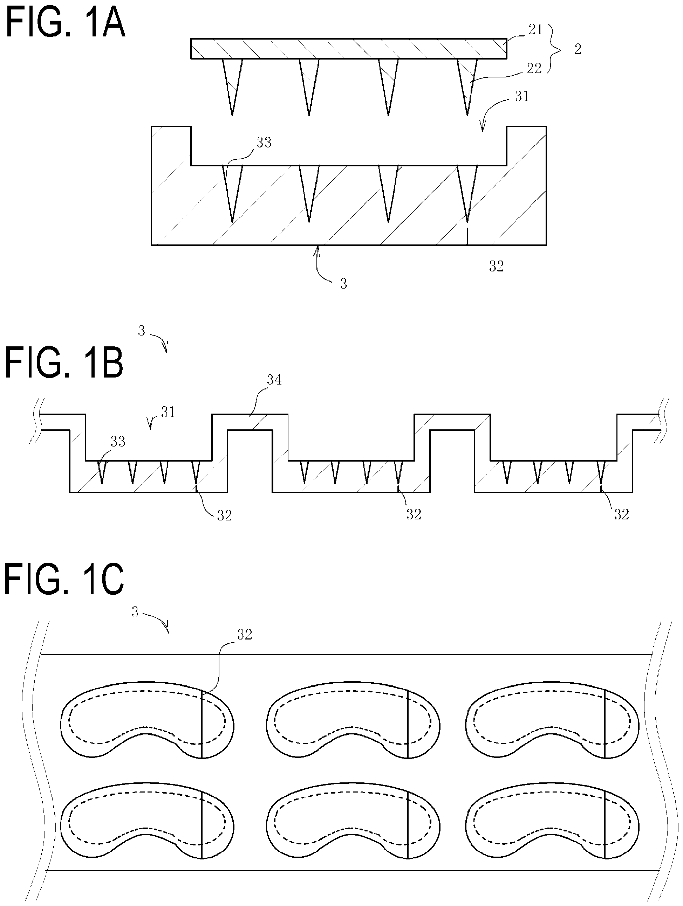

A microneedle sheet 2 includes a sheet-like substrate 21 and a plurality of microneedles 22 protruding from the sheet-like substrate 21 (see FIG. 1A). The plurality of microneedles 22 are arrayed regularly (for example, in a lattice form, a honeycomb form or a staggered form) or irregularly on one face of the sheet-like substrate 21. The sheet-like substrate 21 and the microneedles 22 may be constituted by using different materials, or may be constituted integrally by using a common material. In the present embodiment, as illustrated in FIG. 1A, the sheet-like substrate 21 and the plurality of microneedles 22 are integrally constituted by using a common material. The thickness of the sheet-like substrate 21 may be, for example, not less than 10 µm and not greater than 500 µm, and preferably not less than 20 µm and not greater than 80 µm, such that mechanical strength of the sheet overall can be assured, and the sheet can flexibly deform in conformance with a shape of the skin.

Note that when the sheet-like substrate 21 and the microneedles 22 are constituted by using different materials, the sheet-like substrate 21 is preferably constituted by using a material having flexibility. For example, paper, a resin film or nonwoven fabric can be used.

The microneedles 22 can each have, for example, a round cone shape, a square cone shape, a round truncated cone shape or a volcano shape. The volcano shape refers to a shape in which a side face of a round cone shape or round truncated cone shape is curved inward. The protrusion height of each of the microneedles 22 from the sheet-like substrate 21 can be, for example, not less than 10 µm and not greater than 500 µm. Density of the microneedles 22 can be, for example, not less than 100/cm² and not greater than 500/cm².

The material of the microneedle sheet 2 is preferably a material containing a water-soluble polymer having at least one of properties of in vivo dissolution and in vivo decomposition. The microneedles 22 are formed by using the material containing the water-soluble polymer having such a property, and accordingly the microneedles 22 having penetrated the skin gradually change over time due to action of at least one of in vivo dissolution and in vivo decomposition. As a result, the microneedles 22 do not reside for a long time as a solid body in the skin. Furthermore, since the microneedles 22 dissolve due to water content in the skin, it becomes easy to introduce a target substance into the skin smoothly by using the microneedles 22.

Examples of the water-soluble polymer having at least one of properties of in vivo dissolution and in vivo decomposition and being suitable as the material constituting the microneedle sheet 2 can include pullulan, sodium hyaluronate, dextran, sodium chondroitin sulfate, xanthan gum, tamarind gum, glycogen, chitin, chitosan, agarose, pectin and a carboxyvinyl polymer. These water-soluble polymers may each be used alone, or two or more of these water-soluble polymers may be used as a mixture.

Note that the microneedle sheet 2 may contain other additives such as low-molecular-weight organic compounds, provided that the additives do not affect solubility of the microneedle sheet 2.

A housing sheet 3 is a sheet-like member and includes a housing portion 31 for housing the microneedle sheet 2. Examples of a material of the housing sheet 3 can include resins such as polypropylene, polyethylene and polystyrene. The housing sheet 3 includes a single-layer or multilayer structure made from these resins. The housing portion 31 has a convex shape distending toward the outside of the housing sheet 3 (see FIGS. 1A to 1C). Concave portions 33 having inversed shapes of shapes of the microneedles 22 are formed on a base face of the housing portion 31. Furthermore, a notch 32 is formed in the housing portion 31. The notch may be a straight line or may include a curve, but the notch is preferably a half-cut or a perforation. When such a notch is formed, strength of the housing sheet 3 can be maintained until the microneedle sheet 2 is taken from the housing sheet 3, and the housing sheet 3 can be split easily at the notch when the microneedle sheet 2 is taken from the housing sheet 3. Furthermore, as illustrated in FIG. 1B, the housing sheet 3 may include a plurality of the housing portions 31 by using connecting portions 34. Note that the depth of the housing portion 31 configured to house the microneedle sheet 2 may be the same as the height of the microneedle sheet 2, or may be any depth enabling the housing portion 31 to house the microneedle sheet 2.

Next, an example of a method for producing the housing sheet will be described.

First, a resin sheet including a resin material such as polypropylene, polyethylene and polystyrene is prepared. Next, a housing portion is formed in the resin sheet by using a molding method such as vacuum molding, vacuum pressure molding and cold molding. Next, a mold including protrusions having the same shapes as shapes of the microneedles 22 of the microneedle sheet 2 is pressed into the base face of the housing portion 31. Accordingly, the concave portions 33 having inversed shapes of shapes of the microneedles 22 can be formed. Finally, the notch 32 is formed in the housing portion 31. The notch 32 can be formed by, for example, moving a fixed blade or a rotating blade vertically or by sliding a fixed blade or a rotating blade horizontally against a face in which the notch is to be formed. Furthermore, the notch can also be formed by scanning with a laser. The notch 32 preferably extends toward a tip of at least one of the concave portions 33 (see FIGS. 1A, 1B). Accordingly, when the housing sheet 3 is split at the notch 32 to take the microneedle sheet 2, stress is concentrated at the tips of the concave portions 33 and it becomes easy to split the housing sheet 3. The housing sheet 3 may include one resin sheet in which a plurality of the housing portions 31 are formed (see FIGS. 1B, 1C), or may include a sheet split into individual pieces by cutting the connecting portions 34 (see FIG. 1A). However, as illustrated in FIG. 1C, the housing sheet 3 including one resin sheet in which the plurality of housing portions 31 are formed is preferred because the notch 32 is easy to form at the same location of each of the housing portions 31. Note that a plurality of the notches 32 may be formed in the housing portion 31.

Accordingly, the housing sheet of the present invention can be obtained.

Next, an example of a method for taking the microneedle sheet from the housing sheet of the present invention will be described.

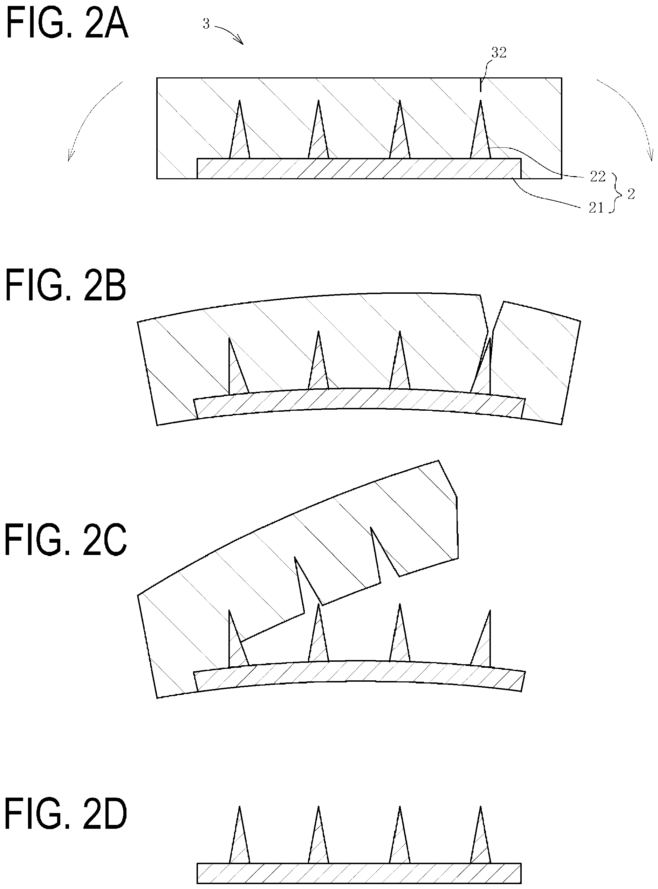

A user of the microneedle sheet 2 holds the housing sheet 3 housing the microneedle sheet, with the notch 32 facing upward (see FIG. 2A). Next, the user bends the housing sheet 3 in directions of arrows in FIG. 2A, and splits the housing sheet 3 at the notch 32. Since the microneedle sheet 2 is flexible, the housing sheet 3 can easily bend. Owing to recoil due to the splitting, the housing sheet near the notch 32 rises up slightly from the microneedle sheet 2 (see FIG. 2B). The user holds a portion rising up, and slowly peels the split housing sheet. Subsequently, the user holds another portion rising up of the split housing sheet to slowly peel the split housing sheet, and accordingly the user can take the microneedle sheet 2 (see FIGS. 2C, 2D). That is, according to the housing sheet of the present invention, when the microneedle sheet is taken, a portion or all of the microneedles do not remain on the housing sheet side.

Note that the above example describes the embodiment in which the housing sheet 3 is peeled with the notch 32 facing upward, but the housing sheet 3 may be peeled with the notch 32 facing downward.

Next, an example of a microneedle sheet package according to an embodiment of the present invention will be described.

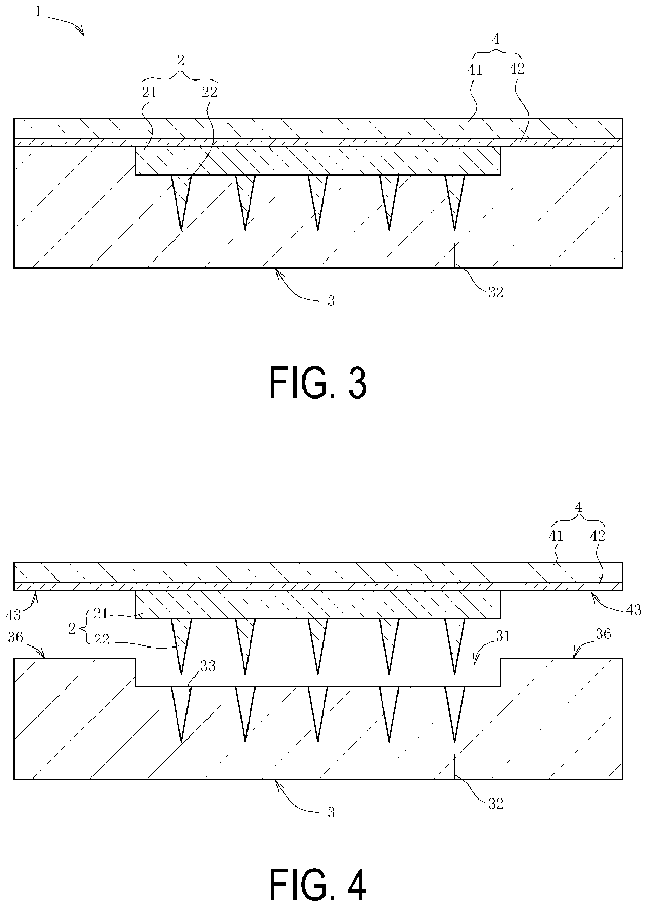

The microneedle sheet package of the present invention includes a microneedle sheet in which a plurality of microneedles are disposed on one face of a sheet-like substrate, a sticky sheet stuck entirely to the other face of the sheet-like substrate and including a first excess portion jutting out from the sheet-like substrate along a peripheral shape of the sheet-like substrate, and a housing sheet including a housing portion in which concave portions having inverted shapes of shapes of the microneedles are formed, and in which the microneedle sheet is housed and in which a notch is formed, and including a face stuck to the first excess portion (see FIG. 3, FIG. 4).

Since a microneedle sheet 2 is similar to the microneedle sheet described above, description of the microneedle sheet 2 will be omitted.

A sticky sheet 4 includes a support sheet 41 and a sticky agent layer 42 formed on one face of the support sheet 41. A shape of the sticky sheet includes a first excess portion 43 jutting out from a sheet-like substrate 21 along a peripheral shape of the sheet-like substrate 21. That is, the sticky sheet is larger in area than the sheet-like substrate 21, and peripheral shapes of the sticky sheet and the sheet-like substrate 21 are similar. The sticky sheet 4 is entirely stuck via the sticky agent layer 42 to the other face of the sheet-like substrate 21, that is, a face on which microneedles 22 are not formed. Therefore, when the sticky sheet 4 and the sheet-like substrate 21 are stuck together, the first excess portion 43 jutting out from the sheet-like substrate 21 can be formed on the sticky sheet 4. Since the first excess portion 43 includes the sticky agent layer 42, after the microneedle sheet 2 to which the sticky sheet 4 is stuck is taken from a housing sheet 3, the microneedle sheet 2 can be stuck to the skin of a user by the sticky agent layer 42 of the first excess portion 43.

The housing sheet 3 of the present embodiment differs from the housing sheet described above in that the housing sheet 3 includes a face 34 stuck to the first excess portion. The face 34 stuck to the first excess portion may have the same height as or a different height from the height of a face of the sheet-like substrate 22 stuck to the sticky agent layer 42 when the microneedle sheet 2 is housed in a housing portion 31. However, when a microneedle sheet package 1 is produced by housing the microneedle sheet 2 in the housing portion 31 and subsequently sticking the sticky sheet 4, the face 34 stuck to the first excess portion preferably has the same height as the height of the face of the sheet-like substrate 22 stuck to the sticky agent layer 42 when the microneedle sheet 2 is housed in the housing portion 31. Accordingly, when the microneedle sheet 2 is housed in the housing portion 31, no step is formed between the face of the sheet-like substrate 22 stuck to the sticky agent layer 42 and the face 34 stuck to the first excess portion. Therefore, when the sticky sheet is stuck, sticking force is propagated without dispersing, and as a result, the sticky sheet 4 can be fixed securely to the housing sheet 3 and the microneedle sheet 2.

An example of a method for producing the microneedle sheet package of the present embodiment will be described below.

First, a raw material solution of the microneedle sheet is prepared. The raw material solution contains a water-soluble polymer having at least one of properties of in vivo dissolution and in vivo decomposition and being suitable as a material constituting the microneedle sheet 2. Examples of such a water-soluble polymer can include pullulan, sodium hyaluronate, dextran, sodium chondroitin sulfate, xanthan gum, tamarind gum, glycogen, chitin, chitosan, agarose, pectin and a carboxyvinyl polymer. The raw material solution is obtained by dissolving the water-soluble polymer in a solvent. For example, water or a mixed solvent of water and alcohol can be used for the solvent.

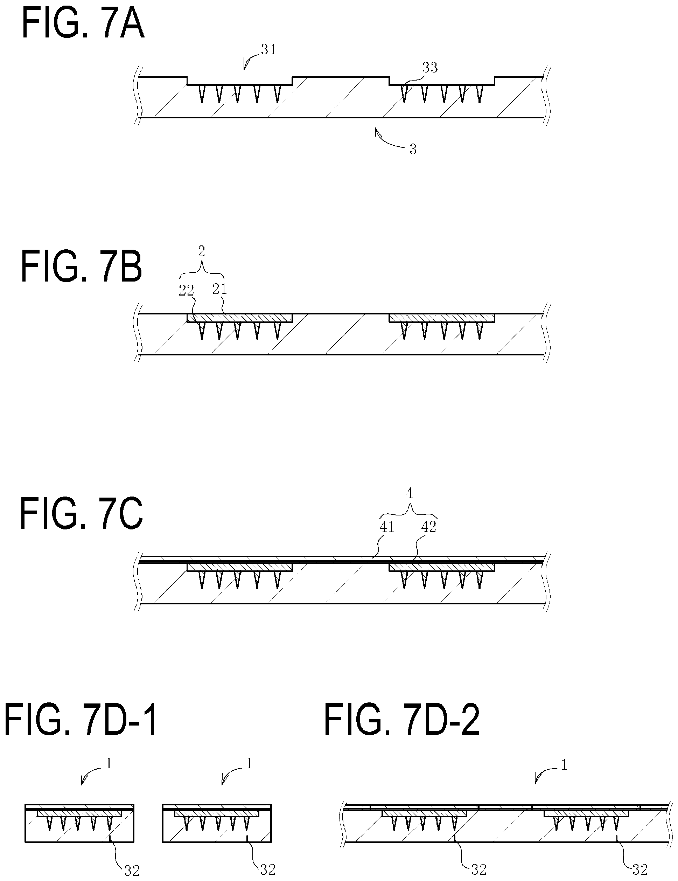

Next, the housing sheet 3 including a plurality of concave portions 33 formed on a base face of the housing portion 31 is prepared (see FIG. 7A). The concave portions 33 are arrayed regularly (for example, in a lattice form, a honeycomb form or a staggered form) or irregularly on one face of the housing sheet 3. The concave portions 33 are formed to each assume an inversed shape of, for example, a round cone shape, a square cone shape, a round truncated cone shape or a volcano shape. The depth of each of the concave portions 33 can be, for example, not less than 10 µm and not greater than 500 µm, and preferably not less than 50 µm and not greater than 200 µm. Furthermore, the concave portions 33 are preferably provided at density of, for example, not less than 100/cm² and not greater than 500/cm².

Next, the raw material solution is injected into the housing portion 31 by using, for example, a dispenser, a dispensing machine, a squeegee or an injector, and is supplied to reach the same height as the height of an inner wall of the housing portion 31 (see FIG. 7B). At this time, high pressure greater than atmospheric pressure may be applied to the top face side of the housing sheet 3 to facilitate filling of the concave portions 33 with the raw material solution.

The raw material solution filling the housing portion 31 is subjected to drying, and accordingly the microneedle sheet 2 can be produced (see FIG. 7B). Next, the sticky sheet 4 is stuck entirely to a face of the housing sheet 3 from a face of the sheet-like substrate 21 opposite a face on which the microneedles 22 are formed (see FIG. 7C). Then, the notch 32 is formed on a face opposite a face of the housing sheet 3 on which the sticky sheet 4 was stuck, and the housing sheet is cut by using, for example, a punch die to obtain an individual microneedle sheet package 1 (see FIG. 7D-1). Note that the notch 32 may be formed after the individual microneedle sheet package 1 is obtained, or may be formed before the filling with the raw material of the microneedle sheet.

Note that as illustrated in FIG. 7D-2, only the sticky sheet may be punched into a shape formed by enlargement of the peripheral shape of the sheet-like substrate 21, and the notch 32 is formed to obtain the microneedle sheet package 1 as a sheet-like microneedle sheet package.

Accordingly, the microneedle sheet package according to the embodiment of the present invention can be obtained.

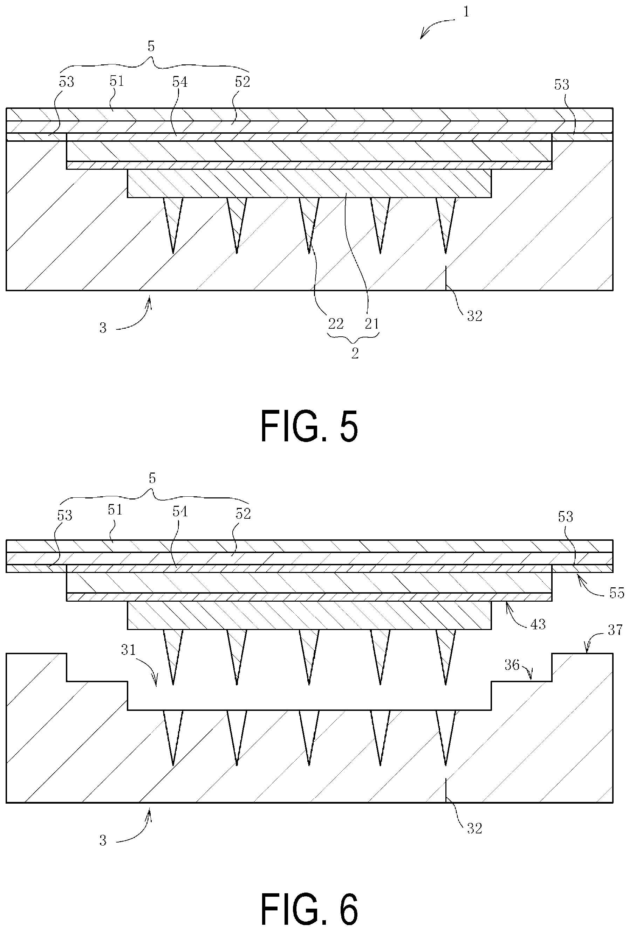

Furthermore, a microneedle sheet package according to another embodiment of the present invention includes a microneedle sheet in which a plurality of microneedles are disposed on one face of a sheet-like substrate; a sticky sheet stuck entirely to the other face of the sheet-like substrate, and including a first excess portion jutting out from the sheet-like substrate along a peripheral shape of the sheet-like substrate; a sealing sheet stuck entirely to a face opposite a face of the sticky sheet to which the sheet-like substrate is stuck, and including a second excess portion jutting out from the sticky sheet along a peripheral shape of the sticky sheet; and a housing sheet including a housing portion in which concave portions having inverted shapes of shapes of the microneedles are formed, and in which the microneedle sheet is housed and in which a notch is formed, and including a face stuck to the first excess portion and a face stuck to the second excess portion (see FIGS. 5, 6).

The microneedle sheet package of this embodiment differs from the microneedle sheet package described with reference to FIGS. 3 and 4 in that a housing sheet 3 includes a face 35 stuck to the second excess portion, and in that the microneedle sheet package of this embodiment includes a sealing sheet 5.

The face 35 stuck to the second excess portion may have the same height as or a different height from the height of a face opposite a face of a support sheet 41 on which a sticky agent layer 42 is formed when a microneedle sheet 2 with a sticky sheet 4 is housed in a housing portion 31. However, when a microneedle sheet package 1 is produced by housing the microneedle sheet 2 with the sticky sheet 4 in the housing portion 31 and subsequently sticking the sealing sheet 5, the face 35 stuck to the second excess portion preferably has the same height as the height of the face opposite the face of the support sheet 41 on which the sticky agent layer 42 is formed when the microneedle sheet 2 with the sticky sheet 4 is housed in the housing portion 31. Accordingly, when the microneedle sheet 2 with the sticky sheet 4 is housed in the housing portion 31, no step is formed between the face opposite the face of the support sheet 41 on which the sticky agent layer 42 is formed and the face 35 stuck to the second excess portion. Therefore, when the sealing sheet 5 is stuck to the housing sheet 3 and the sticky sheet 4, sticking force is propagated without dispersing, and as a result, the sealing sheet 5 can be fixed securely to the housing sheet 3 and the sticky sheet 4. In the microneedle sheet package 1 obtained by sticking the sealing sheet 5 to the housing sheet 3 and the sticky sheet 4, an interior of the housing portion 31 is preferably empty. Accordingly, a sterile state is maintained, and as a result, there is no degradation of the microneedle sheet and the microneedle sheet can be stored cleanly.

The sealing sheet 5 includes a metal layer 52 laminated on a resin layer 51, a sticky layer 54 laminated on a face stuck to the sticky sheet 4, and an adhesive layer 53 laminated on a second excess portion 55. Information such as letters and graphics can be printed on a face of the resin layer 51 opposite a face on which the metal layer 52 is laminated. Furthermore, the sealing sheet 5 also functions to protect the metal layer 52. The metal layer 52 is formed from a material including aluminum or an alloy thereof, and has a high moisture-proof property.

A shape of the sealing sheet includes the second excess portion 55 jutting out from the sticky sheet 4 along a peripheral shape of the sticky sheet 4. That is, the sealing sheet is larger in area than the sticky sheet 4, and peripheral shapes of the sealing sheet and the sticky sheet 4 are similar. The sealing sheet 5 is entirely stuck via the sticky layer 54 to a face opposite a face of the sticky sheet 4 on which the microneedle sheet is stuck. Therefore, when the sealing sheet 4 and the sticky sheet 4 are stuck together, the second excess portion 55 jutting out from the sticky sheet 4 can be formed on the sealing sheet 5. The adhesive layer 53 is formed on the second excess portion 55. The sealing sheet 5 and the housing sheet 3 can be fixed by the adhesive layer 53.

An example of a method for producing the microneedle sheet package according to another embodiment of the present invention will be described below.

First, a raw material solution of the microneedle sheet is prepared. The raw material solution contains a water-soluble polymer having at least one of properties of in vivo dissolution and in vivo decomposition and being suitable as a material constituting the microneedle sheet 2. Examples of such a water-soluble polymer can include pullulan, sodium hyaluronate, dextran, sodium chondroitin sulfate, xanthan gum, tamarind gum, glycogen, chitin, chitosan, agarose, pectin and a carboxyvinyl polymer. The raw material solution is obtained by dissolving the water-soluble polymer in a solvent. For example, water or a mixed solvent of water and alcohol can be used for the solvent.

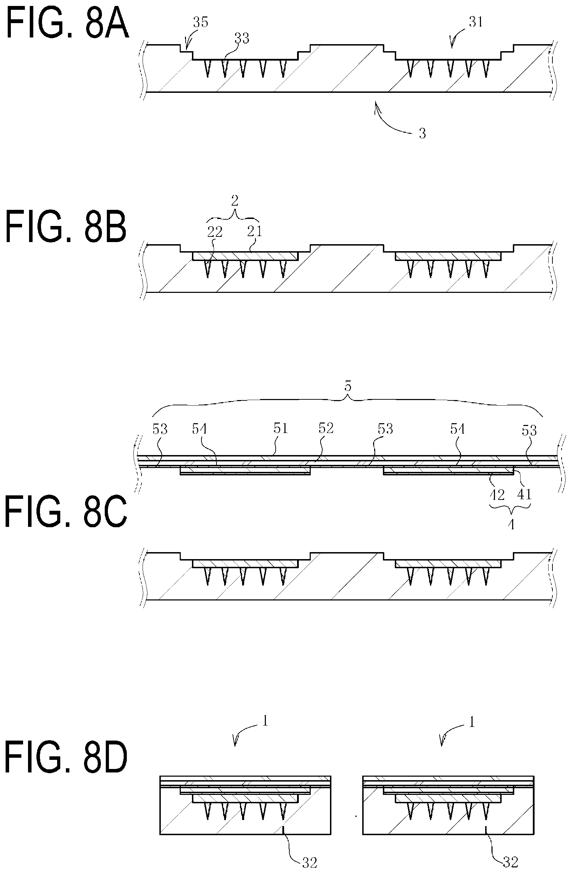

Next, the housing sheet 3 including a plurality of concave portions 33 formed on a base face of the housing portion 31 and including a step portion 35 formed on an inner wall of the housing portion 31 is prepared (see FIG. 8A). The concave portions 33 are arrayed regularly (for example, in a lattice form, a honeycomb form or a staggered form) or irregularly on one face of the housing sheet 3. The concave portions 33 are formed to each assume an inversed shape of, for example, a round cone shape, a square cone shape, a round truncated cone shape or a volcano shape. The depth of each of the concave portions 33 can be, for example, not less than 10 µm and not greater than 500 µm, and preferably not less than 50 µm and not greater than 200 µm. Furthermore, the concave portions 33 are preferably provided at density of, for example, not less than 100/cm² and not greater than 500/cm².

Next, the raw material solution is injected into the housing portion 31 by using, for example, a dispenser, a dispensing machine, a squeegee or an injector, and is supplied to reach the same height as the height of the step portion 35 (see FIG. 8B). At this time, high pressure greater than atmospheric pressure may be applied to the top face side of the housing sheet 3 to facilitate filling of the concave portions 33 with the raw material solution.

The raw material solution filling the housing portion 31 is subjected to drying, and accordingly the microneedle sheet 2 can be produced (see FIG. 8B). Next, the sealing sheet 5 stuck to the sticky sheet 4 is stuck to the housing sheet 3 and the sheet-like substrate 21 from a face opposite a face of the sheet-like substrate 21 on which the microneedles 22 are formed (see FIG. 8C). Then, the notch 32 is formed on a face opposite a face of the housing sheet 3 on which the sticky sheet 4 and the sealing sheet 5 are stuck, and the housing sheet is cut using, for example, a punch die to obtain an individual microneedle sheet package 1 (see FIG. 8D). The notch 32 may be formed after the individual microneedle sheet package 1 is obtained.

Accordingly, the microneedle sheet package according to another embodiment of the present invention can be obtained.

Next, an example of a method for taking the microneedle sheet from the microneedle sheet package of the present invention will be described.

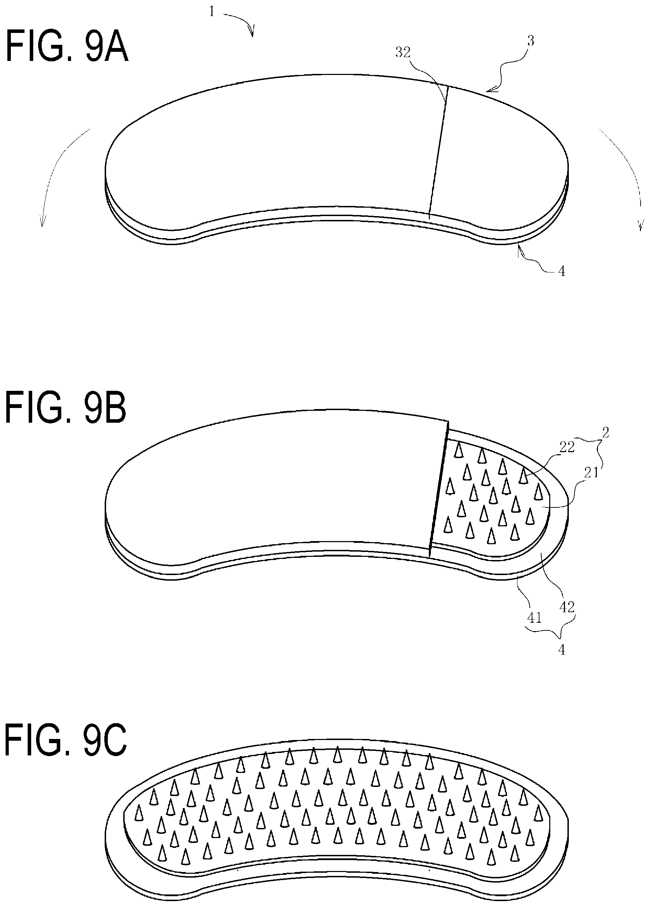

A user of the microneedle sheet holds the microneedle sheet package 1 with the notch 32 facing upward (see FIG. 9A). Next, the user bends the microneedle sheet package 1 in directions of arrows in FIG. 9A, and splits the housing sheet 3 at the notch 32. Since the microneedle sheet 2 and the sticky sheet 4 are flexible, the microneedle sheet package 1 can easily bend. Owing to recoil due to the splitting, the housing sheet near the notch 32 rises up slightly from the sticky agent layer 42 of the sticky sheet 4. The user holds a portion rising up, and slowly peels the split housing sheet (see FIG. 9B). Subsequently, the user holds another portion rising up of the split housing sheet to slowly peel the split housing sheet, and accordingly the user can take the microneedle sheet 2 as a patch-like microneedle sheet to which the sticky sheet 4 is stuck. Therefore, according to the microneedle sheet of the present invention, when the microneedle sheet is taken, at least a portion of the microneedles or the microneedle sheet do not remain on the housing sheet side.

Note that the above example describes the embodiment in which the housing sheet 3 is peeled with the notch 32 facing upward, but the housing sheet 3 may be peeled with the notch 32 facing downward.

Next, another example of a method for taking the microneedle sheet will be described.

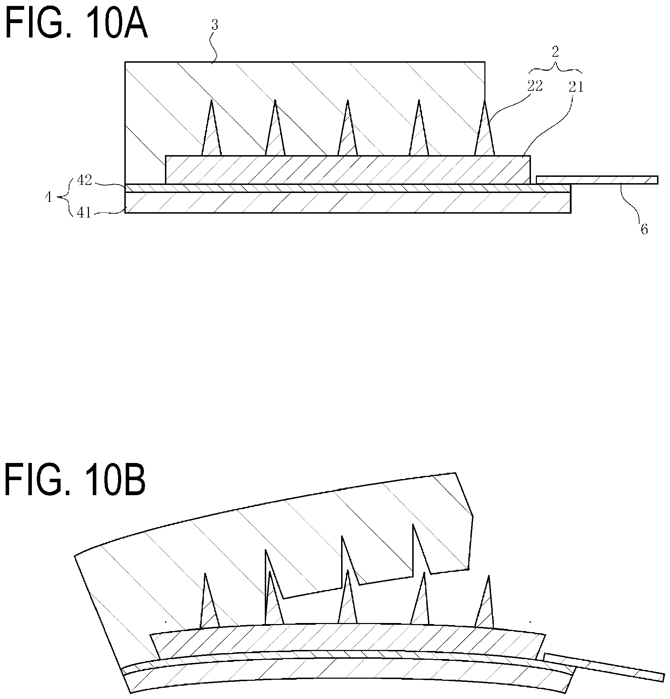

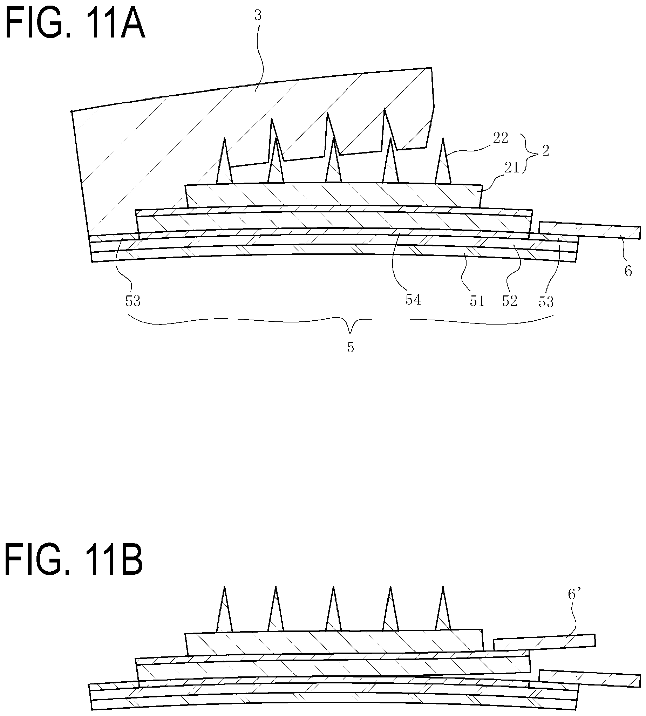

As illustrated in FIG. 9B, a grip portion 6 is stuck to the sticky agent layer 42 in a state where one housing sheet is peeled off (see FIG. 10A). A user can peel off the housing sheet by holding the grip portion 6 together with another housing sheet to pull the grip portion 6 downward in FIG. 10A and pull the housing sheet upward in FIG. 10A. The grip portion 6 is provided, and accordingly the user does not touch the sticky agent layer 42. Therefore, the patch-like microneedle sheet can be stuck to the skin of the user in a state where the sticky agent layer 42 is clean. In a case where there is the sealing sheet 5, the grip portion 6 is stuck to the adhesive layer 53 and the housing sheet is peeled (see FIG. 11A), and subsequently a grip portion 6' is stuck to the sticky agent layer 42 (see FIG. 11B). The user can peel off the sealing sheet 5 by holding the grip portion 6 and the grip portion 6' to pull the grip portion 6 downward in FIG. 11B and pull the grip portion 6' upward in FIG. 11B (see FIG. 11B). Therefore, according to the microneedle sheet of the present invention, when the microneedle sheet is taken, at least a portion of the microneedles or the microneedle sheet do not remain on the housing sheet side.

Accordingly, the microneedle sheet can be taken from the microneedle sheet package of the present invention to be used.

### Reference Signs List

1 Microneedle sheet package
2 Microneedle sheet
21 Sheet-like substrate
22 Microneedle
3 Housing sheet
31 Housing portion
32 Notch
33 Concave portion
34 Connecting portion
35 Step portion
4 Sticky sheet
41 Support sheet
42 Sticky agent layer
43 First excess portion
5 Sealing sheet
51 Resin layer
52 Metal layer
53 Adhesive layer
54 Sticky layer
55 Second excess portion
6, 6' Grip portion
7 Microneedle sheet
71 Sheet-like substrate
72 Microneedle
8 Housing sheet
9 Sticky sheet
10 Sealing sheet

## Claims

1. A housing sheet (3) comprising
a housing portion (31) for housing a microneedle sheet (2) in which a plurality of microneedles (22) are disposed on one face of a sheet-like substrate (21), wherein the housing sheet (3) further comprises:
concave portions (33) having inverted shapes of shapes of the microneedles formed in the housing portion (31), **characterised in that** the housing portion (31) includes a notch (32).

2. The housing sheet (3) according to claim 1, wherein
the notch (32) is a half-cut or a perforation.

3. A microneedle sheet package (1) comprising:
a microneedle sheet (2) in which a plurality of microneedles (22) are disposed on one face of a sheet-like substrate (21);
a sticky sheet (4) stuck entirely to the other face of the sheet-like substrate (21), and including a first excess portion (42) jutting out from the sheet-like substrate (21) along a peripheral shape of the sheet-like substrate (21); and
the housing sheet (3) of claim1, in which the microneedle sheet (2) is housed and in which a notch (32) is formed, and including a face stuck to the first excess portion (43).

4. The microneedle sheet package (1) according to claim 3, further comprising:
a sealing sheet (5) stuck entirely to a face opposite a face of the sticky sheet (4) to which the sheet-like substrate (21) is stuck, and including a second excess portion (55) jutting out from the sticky sheet (4) along a peripheral shape of the sticky sheet (4);
wherein the housing sheet further includes a face stuck to the second excess portion (55).

5. The microneedle sheet package (1) according to claim 4, wherein
the sealing sheet (5) includes
a metal layer (52) laminated on a resin layer (51),
a sticky layer (54) laminated on a face stuck to the sticky sheet (4), and
an adhesive layer (53) laminated on the second excess portion (55).

6. The microneedle sheet package (1) according any one of claims 3 to 5, wherein
the notch (32) is a half-cut or a perforation.

7. The microneedle sheet package (1) according any one of claims 4 to 6, wherein
an interior of the housing portion (31) is empty.

## Patentansprüche

1. Gehäusefolienbahn (3), umfassend
einen Gehäuseabschnitt (31) zum Aufnehmen einer Mikronadelfolienbahn (2), in der eine Vielzahl von Mikronadeln (22) auf einer Fläche eines folienbahnartigen Substrats (21) angeordnet ist, wobei
die Gehäusefolienbahn (3) ferner umfasst:
konkave Abschnitte (33) mit umgekehrten Formen von Formen der Mikronadeln, die in dem Gehäuseabschnitt (31) ausgebildet sind, **dadurch gekennzeichnet, dass**
der Gehäuseabschnitt (31) eine Kerbe (32) einschließt.

2. Gehäusefolienbahn (3) nach Anspruch 1, wobei
die Kerbe (32) ein Halbschnitt oder eine Perforation ist.

3. Mikronadelfolienbahnverpackung (1), umfassend:
eine Mikronadelfolienbahn (2), in der eine Vielzahl von Mikronadeln (22) auf einer Fläche eines folienbahnartigen Substrats (21) angeordnet ist;
eine Klebefolienbahn (4), die vollständig an der anderen Fläche des folienbahnartigen Substrats (21) klebt und einen ersten Überstandabschnitt (42) umfasst, der von dem folienbahnartigen Substrat (21) entlang einer Umfangsform des folienbahnartigen Substrats (21) hervorsteht; und
die Gehäusefolienbahn (3) nach Anspruch 1, in der die Mikronadelfolienbahn (2) untergebracht ist und in der eine Kerbe (32) ausgebildet ist, und einschließlich einer Seite, die an dem ersten Überstandabschnitt (43) klebt.

4. Mikronadelfolienbahnverpackung (1) nach Anspruch 3, ferner umfassend:
eine Versiegelungsfolienbahn (5), die vollständig an einer Fläche gegenüber einer Fläche der Klebefolienbahn (4) klebt, an der das folienbahnartige Substrat (21) klebt, und einschließlich eines zweiten Überstandabschnitts (55), der von der Klebefolienbahn (4) entlang einer Umfangsform der Klebefolienbahn (4) hervorsteht;
wobei die Gehäusefolienbahn ferner eine Fläche einschließt, die an dem zweiten Überstandabschnitt (55) klebt.

5. Mikronadelfolienbahnverpackung (1) nach Anspruch 4, wobei die Versiegelungsfolienbahn (5) einschließt
eine auf eine Harzschicht (51) laminierte Metallschicht (52),
eine klebrige Schicht (54), die auf eine Seite laminiert ist, die an der Klebefolienbahn (4) klebt, und
eine Klebeschicht (53), die auf den zweiten Überstandabschnitt (55) laminiert ist.

6. Mikronadelfolienbahnverpackung (1) nach einem der Ansprüche 3 bis 5, wobei die Kerbe (32) ein Halbschnitt oder eine Perforation ist.

7. Mikronadelfolienbahnverpackung (1) nach einem der Ansprüche 4 bis 6, wobei ein Inneres des Gehäuseabschnitts (31) leer ist.

## Revendications

1. Feuille de boîtier (3) comprenant :
une partie de boîtier (31) destinée à loger une feuille de micro-aiguilles (2) dans laquelle une pluralité de micro-aiguilles (22) sont disposées sur une face d'un substrat de type feuille (21), dans laquelle la feuille de boîtier (3) comprend en outre :
des parties concaves (33) ayant des formes inversées de formes des micro-aiguilles formées dans la partie de boîtier (31), **caractérisée en ce que** la partie de boîtier (31) comporte une encoche (32).

2. Feuille de boîtier (3) selon la revendication 1, dans laquelle l'encoche (32) est une demi-coupe ou une perforation.

3. Emballage de feuille de micro-aiguilles (1) comprenant :
une feuille de micro-aiguilles (2) dans laquelle une pluralité de micro-aiguilles (22) sont disposées sur une face d'un substrat de type feuille (21) ;
une feuille collante (4) collée entièrement à l'autre face du substrat de type feuille (21), et comprenant une première partie en excès (42) s'étendant hors du substrat de type feuille (21) le long d'une forme périphérique du substrat de type feuille (21) ; et
la feuille de boîtier (3) selon la revendication 1, dans laquelle la feuille de micro-aiguilles (2) est logée et dans laquelle une encoche (32) est formée, et comportant une face collée à la première partie en excès (43).

4. Emballage de feuille de micro-aiguilles (1) selon la revendication 3, comprenant en outre :
une feuille d'étanchéité (5) collée entièrement à une face opposée à une face de la feuille collante (4) à laquelle le substrat de type feuille (21) est collé, et comprenant une deuxième partie en excès (55) s'étendant hors de la feuille collante (4) le long d'une forme périphérique de la feuille collante (4) ;
dans lequel la feuille de boîtier comprend en outre une face collée à la deuxième partie en excès (55).

5. Emballage de feuille à micro-aiguilles (1) selon la revendication 4, dans lequel la feuille d'étanchéité (5) comprend
une couche métallique (52) stratifiée sur une couche de résine (51),
une couche collante (54) stratifiée sur une face collée à la feuille collante (4), et
une couche adhésive (53) stratifiée sur la deuxième partie en excès (55).

6. Emballage de feuille à micro-aiguilles (1) selon l'une quelconque des revendications 3 à 5, dans lequel l'encoche (32) est une demi-coupe ou une perforation.

7. Emballage de feuille à micro-aiguilles (1) selon l'une quelconque des revendications 4 à 6, dans lequel un intérieur de la partie de boîtier (31) est vide.
